# EUROPEAN PATENT APPLICATION

(11) **EP 1 695 957 A1**
(43) Date of publication of application: **30.08.2006**
(21) Application number: 05004183.9
(22) Date of filing: 25.02.2005
(51) Int. Cl.: C07C 217/54, A61K 31/135

(54) **Crystalline forms of (1RS,3RS,6RS)-6-Dimethylaminomethyl-1-(3-methoxy-phenyl)cyclohexane-1,3-diol hydrochloride**

(71) Applicant: Grünenthal GmbH, 52078 Aachen (DE)
(72) Inventor: Buschmann, Helmut, 08960 Sant Just Desvern (Barcelona) (ES); Hell, Wolfgang, 52066 Aachen (DE); Gruss, Michael, 52080 Aachen (DE); Fischer, Andreas, 52393 Hürtgenwald (DE); Lischke, Dagmar, 52249 Eschweiler (DE)

(57) **Abstract**

This invention relates to solid crystalline forms of (1 RS,3RS,6RS)-6-Dimethylaminomethyl-1-(3-methoxy-phenyl)cyclohexane-1,3-diol hydrochloride (1), methods of producing 1, methods of use of 1, use of 1 as analgesics and pharmaceutical compositions comprising 1.

## Description

This invention relates to solid crystalline forms of (1RS,3RS,6RS)-6-Dimethylaminomethyl-1-(3-methoxy-phenyl)cyclohexane-1,3-diol hydrochloride **(1),** methods of producing **1,** methods of use of **1,** use of **1** as analgesics and pharmaceutical compositions comprising **1.**

The treatment of pain conditions is of great importance in medicine. There is currently a world-wide need for additional pain therapy. The pressing requirement for a target-oriented treatment of pain conditions which is right for the patient, which is to be understood as the successful and satisfactory treatment of pain for the patients, is documented in the large number of scientific works which have recently and over the years appeared in the field of applied analgesics or on basic research on nociception.

The underlying object of the present invention was to find new solid forms of (1 RS,3RS,6RS)-6-Dimethylaminomethyl-1-(3-methoxy-phenyl)cyclohexane-1,3-diol hydrochloride, preferred useful in the treatment of pain.

US Pat. Nos. RE 37355 E and 5,733,936 as well as European Patent EP 753 506 B1 disclose the substance and the synthesis of (1RS,3RS,6RS)-6-Dimethylaminomethyl-1-(3-methoxy-phenyl)cyclohexane-1,3-diol hydrochloride in example 18.

It has now been surprisingly found that (1RS,3RS,6RS)-6-Dimethylaminomethyl-1-(3-methoxy-phenyl)cyclohexane-1,3-diol hydrochloride can be produced in several different crystalline forms. The present invention provides the new forms form A, form B, form C, form D and form E of (1RS,3RS,6RS)-6-Dimethylaminomethyl-1-(3-methoxy-phenyl)cyclohexane-1,3-diol hydrochloride. These new forms of (1RS,3RS,6RS)-6-Dimethylaminomethyl-1-(3-methoxy-phenyl)cyclohexane-1,3-diol hydrochloride are useful for producing pharmaceutical compositions.
It is the first time to have access to the relevant polymorphs of this important pharmaceutical compound. The polymorph forms are important for evaluation of synthesis, stability and safety both of this compound and of the pharmaceutical formulation made from this compound. Furthermore each of the polymorph forms are important in terms of the different pharmaceutical formulation process that might be involved, in which each of the polymorphs are advantageous with their special physico-chemical properties.

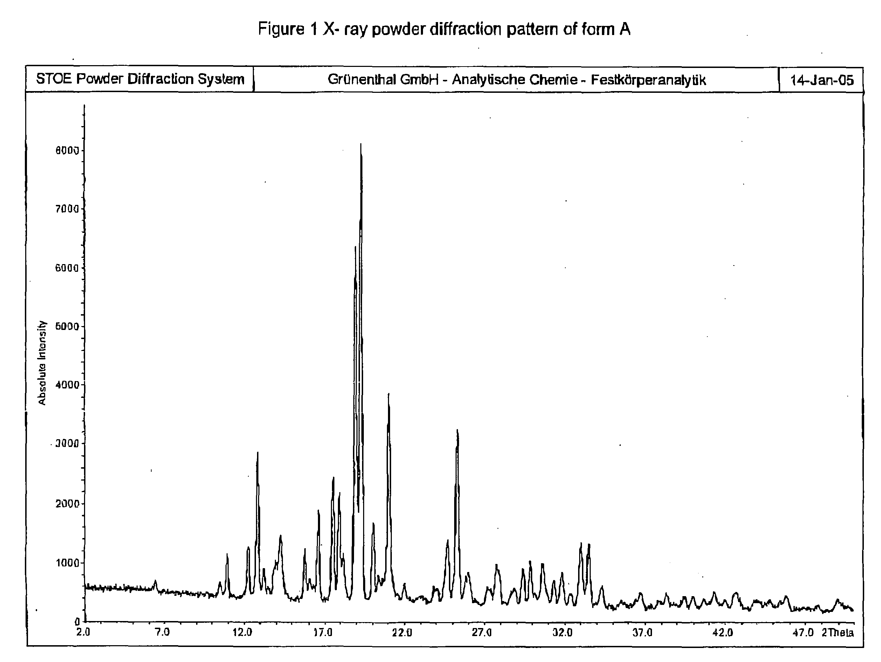

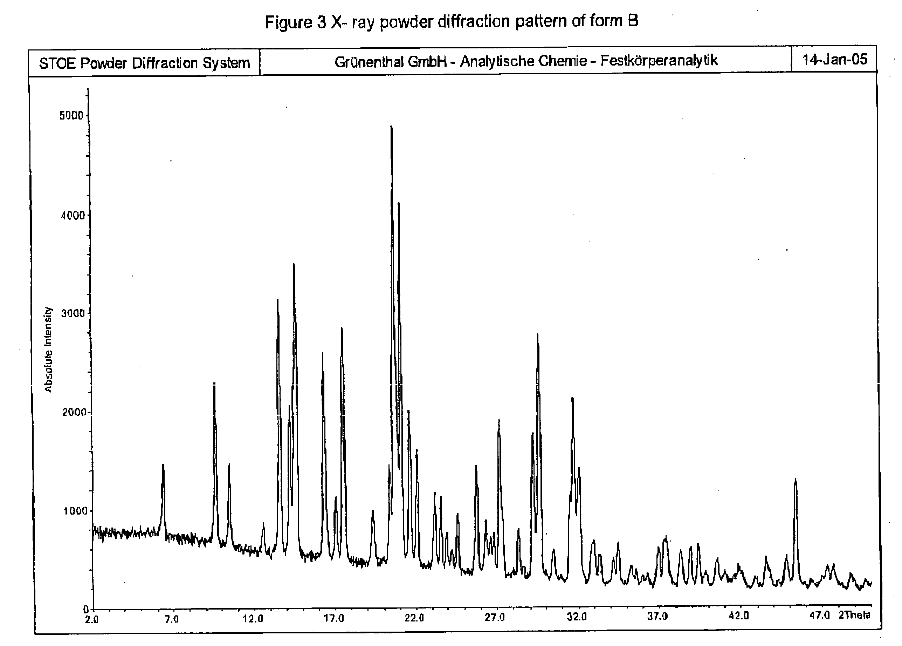

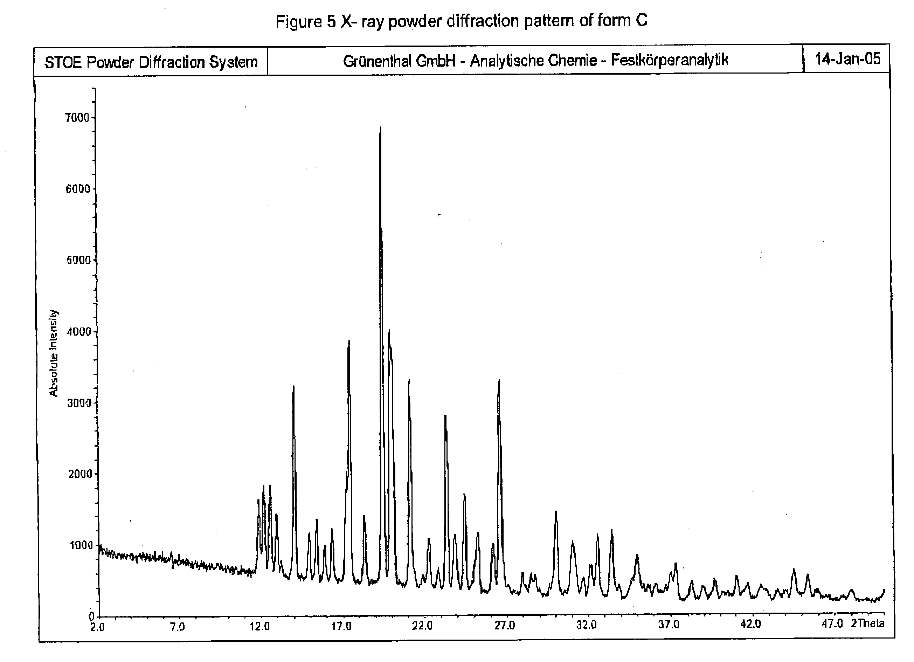

The new crystalline forms can be identified by X-ray powder diffraction. The X-ray powder diffraction ("XRPD") patterns are shown in Figure 1, Figure 3, Figure 5, Figure 7 and Figure 9 with the peak listing shown in Table 1. Ambient temperature and room temperature is defined as 23±3°C.

The most important X-ray lines (2-theta values) in terms of intensity characterizing form A of (1RS,3RS,6RS)-6-Dimethylaminomethyl-1-(3-methoxyphenyl)cyclohexane-1,3-diol hydrochloride showing one or a combination of the following in a powder diffraction measurement when measured using Cu Kα radiation at ambient temperature are 12.9± 0.2, 17.5±0.2, 19.0±0.2, 19.3±0.2, 21.0±0.2 and 25.3±0.2.

To discriminate crystalline form A of (1RS,3RS,6RS)-6-Dimethylaminomethyl-1-(3-methoxy-phenyl)cyclohexane-1,3-diol hydrochloride from the other forms it is more advantageous to look at the unique peaks in the X-ray diffraction diagram, i.e. e. the lines with sufficient intensity at 2-theta values, where the other modifications do not show lines with significant intensity. Such characteristic X-ray lines (2-theta values) for form A in a powder diffraction pattern when measured using CuKα radiation at ambient temperature are: 11.0±0.2, 12.3±0.2, 12.9±0.2, 16.6±0.2, 17.9±0.2, 19.0±0.2 and 25.3±0.2.

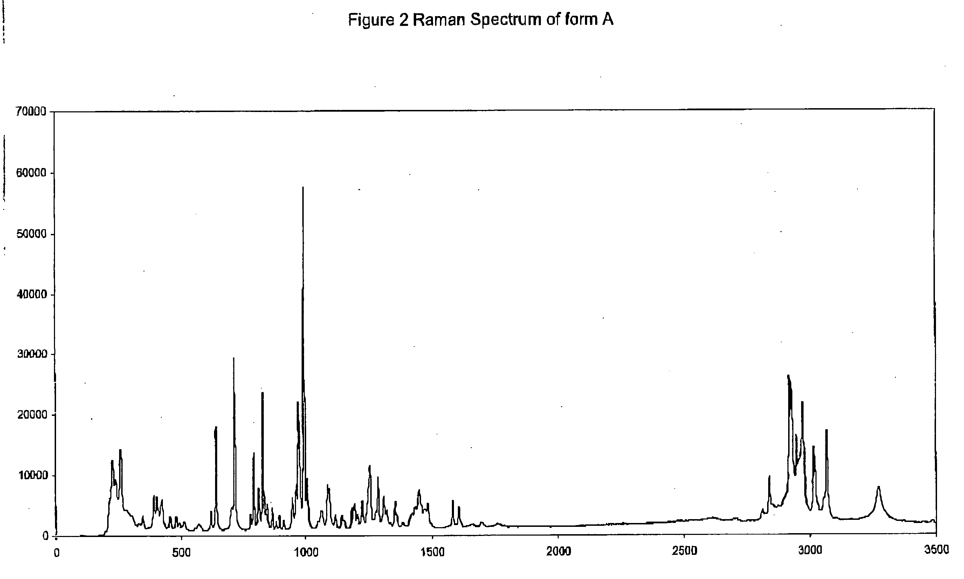

RAMAN technique can also be used to identify the crystalline form A of (1 RS,3RS,6RS)-6-Dimethytaminomethyt-1-(3-methoxy-phenyi)cyclohexane-1,3-diol hydrochloride as shown in Fig. 2. The most important Raman wave numbers (cm⁻¹) in terms of intensity characterizing form A of (1RS,3RS,6RS)-6-Dimethylaminomethyl-1-(3-methoxy-phenyl)cyclohexane-1,3-diol hydrochloride showing one or a combination of the following in a Raman spectrum when measured using a laser wave length of 632 nm are 227 ± 4, 262 ± 4, 643 ± 4, 716 ± 4, 813 ± 4, 830 ± 4, 970 ± 4, 993 ± 4, 1252 ± 4, 2973 ± 4 and 3273 ± 4 cm-¹.

The Invention further relates to processes for the preparation of crystalline form A of (1RS ,3RS,6RS).6.Dimethylaminomethyl.1 .(3.methoxy.phenyl)cyclohexane.1 ,3-diol hydrochloride.

Object of the invention is a Process for production of (1RS,3RS,6RS)-6-Dimethylaminomethyl-1-(3-methoxy-phenyl)cyclohexane-1,3-diol hydrochloride of crystalline form A comprising precipitating the free base solution of (1RS,3RS,6RS)-6-Dimethylaminomethyl-1-(3-methoxy-phenyl)cyclohexane-1,3-diol hydrochloride, steering and filtering, drying of the solid at 40-60 °C, preferred at 45-55°C, and reduced pressure of less than 300 mbar, preferred 150 mbar, for 20-40, preferred 20-28, hours, keeping the temperature at 120-140°C, preferred 125-135°C ,for 60-80, preferred 70-74, hours at less than 150 mbar pressure, reducing the temperature to 50-70, preferred 55-65°C, and drying the product for another 20-60, preferred 20-30, hours at 50-70, preferred 55-65°C, at less than 150 mbar.

The very preferred process starts from a free base solution of (1RS,3RS,6RS)-6-Dimethylaminomethyl-1-(3-methoxy-phenyl)cyclohexane-1,3-diol hydrochloride which is precipitated with concentrated hydrochloric acid and allowed to stir further 24 hours. The liquid phase is filtered off. The solid is dried at 50°C ±5°C and reduced pressure of less than 150 mbar for 24 hours. After that the temperature is kept at 130 °C for another 72 ±10 hours at less than 150 mbar pressure. Reduce the temperature again to 60 °C and dry the product for another 24 hours at 60 °C and less than 150 mbar. The remaining substance is (1 RS,3RS,6RS)-6-Dimethylaminomethyl-1-(3-methoxy-phenyl)cyclohexane-1,3-diol hydrochloride form A.

This invention further relates to a new Crystalline form B of (1RS,3RS,6RS)-6-Dimethylaminomethyl-1-(3-methoxy-phenyl)cyclohexane-1,3-diol hydrochloride. Crystalline form B can be used as active ingredient in pharmaceutical compositions.

Therefore the invention further relates to a pharmaceutical composition containing as active ingredient (1RS,3RS,6RS)-6-Dimethylaminomethyl-1-(3-methoxyphenyl)cyclohexane-1,3-diol hydrochloride of crystalline form B according to the invention and at least one suitable additive and/or auxiliary substance.

The most important X-ray lines (2-theta values) in terms of intensity characterizing form B of (1RS,3RS,6RS)-6-Dimethylaminomethyl-1-(3-methoxyphenyl)cyclohexane-1,3-diol hydrochloride showing one or a combination of the following in a powder diffraction measurement when measured using Cu K_{α} radiation at ambient temperature are 9.7± 0.2, 13.6±0.2, 14.6±0.2, 16.3±0.2, 20.6±0.2 and 29.6±0.2.

To discriminate crystalline form B of (1RS,3RS,6RS)-6-Dimethylaminomethyl-1-(3. methoxy-phenyl)cyclohexane-1,3-diol hydrochloride from the other modifications it is more advantageous to look at the unique peaks in the X-ray diffraction diagram, i.e e. the lines with sufficient intensity at 2-theta values, where the other modifications do not show lines with significant intensity. Such characteristic X-ray lines (2-theta values) for form B in a powder diffraction pattern when measured using CuKα radiation at ambient temperature are: 9.7±0.2, 10.5±0.2, 13.6±0.2, 14.6±0.2 20.6±0.2, 21.6±0.2, 27.2±0.2 and 29.6±0.2.

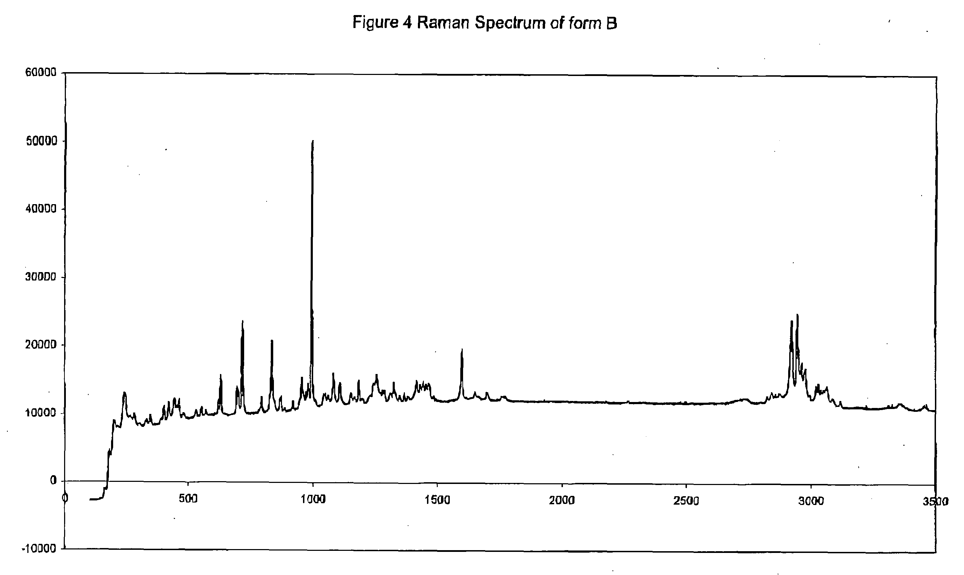

RAMAN technique can also be used to identify of the crystalline form B of (1RS,3RS,6RS)-6-Dimethylaminomethyl-1 -(3-methoxy-phenyl)cyclohexane-1,3-diol hydrochloride as shown in Fig. 4. Especially the range between 800 cm⁻¹ and 200 cm⁻¹ is advantageously used also by way of RAMAN microscopy. The most important Raman wave numbers (cm⁻¹) in terms of intensity characterizing form B of (1RS,3RS,6RS)-6-Dimethylaminomethyl-1 -(3-methoxy-phenyl)cyclohexane-1,3-diol hydrochloride showing one or a combination of the following in a Raman spectrum when measured using a laser wave length of 632 nm are 633 ± 4, 720 ± 4, 836 ± 4, 995 ± 4, 1109 ± 4, 1600 ± 14, 2921 ± 4, and 2944 ± 4 cm⁻¹

The Invention further relates to processes for the preparation of crystalline form B of (1RS,3RS,6RS)-6-Dimethylaminomethyl-1-(3-methoxy-phenyl)cyclohexane-1,3-diot hydrochloride.

Object of the invention is a process for production of (1RS,3RS,6RS)-6-Dimethylaminomethyl-1-(3-methoxy-phenyl)cyclohexane-1,3-diol hydrochloride of crystalline form B comprising the steps dissolving crystalline form A in ethanol and/or water, preferred ratio range ethanol to water 9,5:0,5 to 0,5 to 9,5 , very preferred ethanol and water ratio 9:1, sonication , filtering, and allowing evaporating at room temperature by atmospheric pressure.

The preferred process starts from crystalline form A of (1RS,3RS,6RS)-6-Dimethylaminomethyl-1-(3-methoxy-phenyl)cyclohexane-1,3-diol hydrochloride. In one embodiment of the process (1RS,3RS,6RS)-6-Dimethylaminomethyl-1-(3-methoxy-phenyl)cyclohexane-1,3-diol hydrochloride of crystalline form B is produced by dissolving 30 -50 mg (1RS,3RS,6RS)-6-Dimethylaminomethyl-1-(3-methoxyphenyl)cyclohexane-1,3-diol hydrochloride of crystalline form A in a mixture of about 100 µl ethanol and water (w : w; 9 : 1) of HPLC grade. After sonication to assist dissolution the solution is filtered through a 0.2 micron filter attached to a syringe into a scintillation vial at ambient temperature. The solvent is allowed to evaporate at ambient temperature at athmospheric pressure. The remaining substance is (1RS,3RS,6RS)-6-Dimethylaminomethyl-1-(3-methoxy-phenyl)cyclohexane-1,3-diol hydrochloride form B.

Alternatively instead of an ethanol and water (w : w; 9 : 1) mixture an ethanol and water mixture of w:w; 6:1 and HPLC grade can be used.

Alternatively instead of an ethanol and water (w : w; 9 : 1) mixture water of HPLC grade can be used.

This invention further relates to a new Crystalline form C of (1RS,3RS,6RS)-6-Dimethylaminomethyl-1-(3-methoxy-phenyl)cyclohexane-1,3-diol hydrochloride. Crystalline form C can be used as active ingredient in pharmaceutical compositions.

Therefore the invention further relates to a pharmaceutical composition containing as active ingredient (1RS,3RS,6RS)-6-Dimethylaminomethyl-1-(3-methoxyphenyl)cyclohexane-1,3-diol hydrochloride of crystalline form C according to the invention and at least one suitable additive and/or auxiliary substance.

The most important X-ray lines (2-theta values) in terms of intensity characterizing form C of (1RS,3RS,6RS)-6-Dimethylaminomethyl-1-(3-methoxyphenyl)cyclohexane-1,3-diol hydrochloride showing one or a combination of the following in a powder diffraction measurement when measured using Cu K_{α} radiation at ambient temperature are 14.1±0.2, 17.4±0.2, 19.5±0.2, 20.0±0.2, 23.4±0.2 and 26.6±0.2.

To discriminate crystalline form C of (1RS,3RS,6RS)-6-Dimethylaminomethyl-1-(3-methoxy-phenyl)cyclohexane-1,3-diol hydrochloride from the other modifications it is more advantageous to look at the unique peaks in the X-ray diffraction diagram, i.e. the lines with sufficient intensity at 2-theta values, where the other modifications do not show lines with significant intensity. Such characteristic X-ray lines (2-theta values) for form C in a powder diffraction pattern when measured using CuKα radiation at ambient temperature are: 11.9±0.2, 12.2±0.2, 12.6t0.2, 15.4±0.2, 17.3±0.2, 22.3±0.2, and 23.4±0.2.

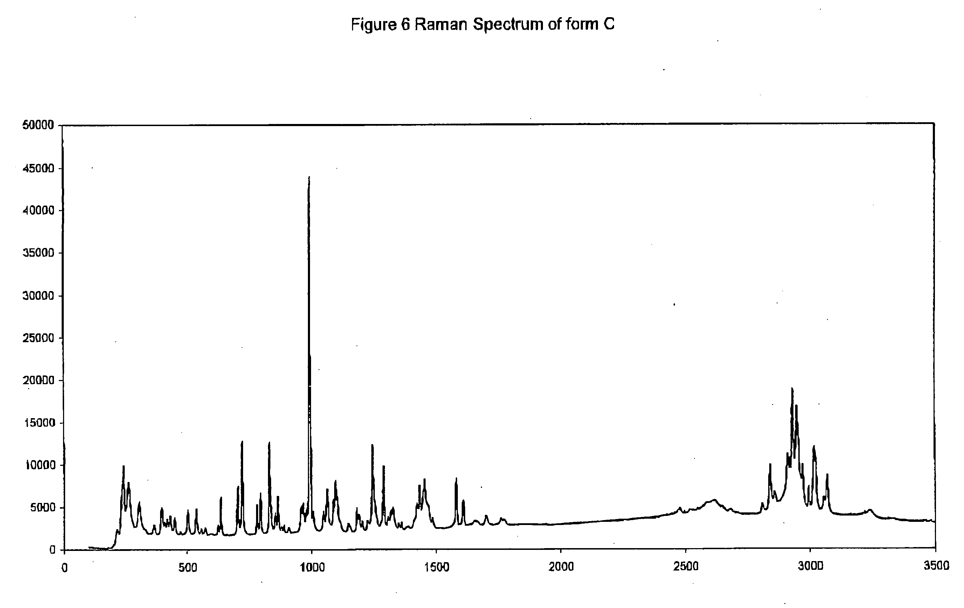

RAMAN technique can also be used to identify of the crystalline form C of (1RS,3RS,6RS).6.Dimethylaminomethyl.1 .(3-methoxy-phenyl)cyclohexane.1 ,3-diol hydrochloride as shown in Fig. 6. The most important Raman wave numbers (cm⁻¹) in terms of intensity characterizing form C of (1RS,3RS,6RS)-6-Dimethylaminomethyl-1-(3-methoxy-phenyl)cyclohexane-1,3-diol hydrochloride showing one or a combination of the following in a Raman spectrum when measured using a laser wave length of 632 nm are 239±4, 305±4, 448±4, 502±4, 537±4, 722±4, 830±4, 992±4, 1094±4, 1243±4, 2928±4 and 2945±4 cm⁻¹.

The Invention further relates to processes for the preparation of crystalline form C of (1RS,3RS,6RS)-6-Dimethylaminomethyl-1 -(3-methoxy-phenyl)cyclohexane-1,3-dio, hydrochloride.

Object of the invention is a process for production of (1RS,3RS,6RS)-6-Dimethylaminomethyl-1-(3-methoxy-phenyl)cyclohexane-1,3-diol hydrochloride of crystalline form C according to claim 15-20 comprising the steps dissolving the free base of (1RS,3RS,6RS)-6-Dimethylaminomethyl-1 -(3-methoxy-phenyl)cydohexane-1,3-diol hydrochloride in isopropanol at 40- xx°C(boiling point of isopropanol), cooling and treatment of solution with hydrogenchloride.

The very preferred process starts from the free base of (1 RS,3RS,6RS)-6-Dimethylaminomethyl-1-(3-methoxy-phenyl)cyclohexane-1,3-diol hydrochloride (GRT3982Y). Approximately 20 g of GRT3982Y were dissolved in isopropanol at an elevated temperature (approximately 45 °C or higher) to reach complete dissolution. The samples were allowed to cool to ambient temperature before treatment with gaseous hydrogen chloride. A constant purge of nitrogen was bubbled through the clear solution while stirring. Anhydrous hydrogen chloride was introduced into the system through the purge stream. Addition of hydrogen chloride was stopped when precipitation was observed. (1RS,3RS,6RS)-6-Dimethylaminomethyl-1-(3-methoxyphenyl)cyclohexane-1,3-diol hydrochloride form C was recovered by vacuum filtration immediately after formation.

This invention further relates to a new Crystalline form D of (1RS,3RS,6RS)-6-Dimethylaminomethyl-1-(3-methoxy-phenyl)cyclohexane-1,3-diol hydrochloride. Crystalline form D can be used as active ingredient in pharmaceutical compositions.

Therefore the invention further relates to a pharmaceutical composition containing as active ingredient (1RS,3RS,6RS)-6-Dimethylaminomethyl-1-(3-methoxyphenyl)cyclohexane-1,3-diol hydrochloride of crystalline form D according to the invention and at least one suitable additive and/or auxiliary substance.

The most important X-ray lines (2-theta values) in terms of intensity characterizing form D of (1RS,3RS,6RS)-6-Dimethylaminomethyl-1-(3-methoxyphenyl)cyclohexane-1,3-diol hydrochloride showing one or a combination of the following in a powder diffraction measurement when measured using Cu K_{α} radiation at ambient temperature are 17.9±0.2, 18.6±0.2, 19.0±0.2, 19.9t0.2 and 25.7±0.2.

To discriminate crystalline form D of (1RS,3RS,6RS)-6-Dimethylaminomethyl-1-(3-methoxy-phenyl)cyclohexane-1,3-diol hydrochloride from the other modifications it is more advantageous to look at the unique peaks in the X-ray diffraction diagram, i.e. the lines with sufficient intensity at 2-theta values, where the other modifications do not show lines with significant intensity. Such characteristic X-ray lines (2-theta values) for form D in a powder diffraction pattern when measured using CuK_{α} radiation at ambient temperature are: 10.3±0.2, 12.7±0.2, 13.0±0.2, 13.5±0.2, 18.6±0.2, 25.7±0.2 and 28.7±0.2:

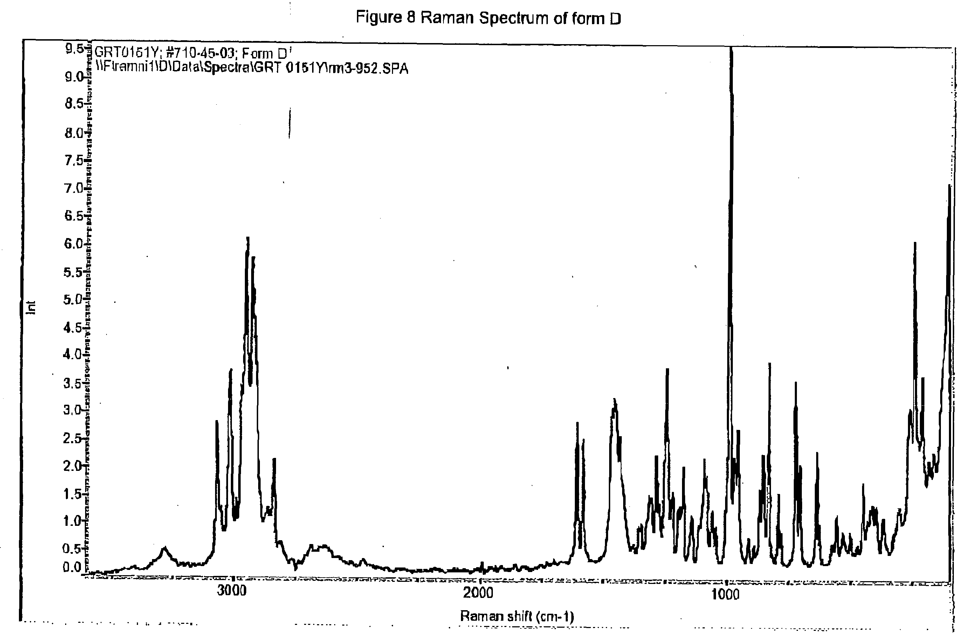

RAMAN technique can also be used to identify of the crystalline form D of (1 RS,3RS,6RS)-6-Dimethylaminomethyl-1-(3-methoxy-phenyl)cyclohexane-1,3-diol hydrochloride as shown in Fig. 8.

The Invention further relates to processes for the preparation of crystalline form D of (1 RS,3RS,6RS)-6-Dimethylaminomethyl-1-(3-methoxy-phenyl)cyclohexane-1,3-diol hydrochloride.

Object of the invention is a process for production of (1RS,3RS,6RS)-6-Dimethylaminomethyl-1-(3-methoxy-phenyl)cyclohexane-1,3-diol hydrochloride of crystalline form D comprising heating form B to 160-185°C, preferred 175-185°C, for 20-50, preferred 30-40 minutes, and cooling to room temperature.

The very preferred process starts from the freshly prepared (1RS,3RS,6RS)-6-Dimethylaminomethyl-1-(3-methoxy-phenyl)cyclohexane-1,3-diol hydrochloride form B. A small sample is heated in a XRPD glass capillary to 180 °C for approx. 35 minutes. After cooling down to ambient temperature (1 RS,3RS,6RS)-6-Dimethylaminomethyl-1-(3-methoxy-phenyl)cyclohexane-1,3-diol hydrochloride form D was recovered.

This invention further relates to a new Crystalline form E of (1RS,3RS,6RS)-6-Dimethylaminomethyl-1-(3-methoxy-phenyl)cyclohexane-1,3-diol hydrochloride. Crystalline form E can be used as active ingredient in pharmaceutical compositions.

Therefore the invention further relates to a pharmaceutical composition containing as active ingredient (1RS,3RS,6RS)-6-Dimethylaminomethyl-1-(3-methoxyphenyl)cyclohexane-1,3-diol hydrochloride of crystalline form E according to the invention and at least one suitable additive and/or auxiliary substance.

The most important X-ray lines (2-theta values) in terms of intensity characterizing form E of (1RS,3RS,6RS)-6-Dimethylaminomethyl-1-(3-methoxyphenyl)cyclohexane-1,3-diol hydrochloride showing one or a combination of the following in a powder diffraction measurement when measured using Cu K_{α} radiation at ambient temperature are 9.7±0.2, 14.9±0.2, 16.6±0.2, 19.2±0.2, 21.4±0.2 and 27.3t0.2.

To discriminate crystalline form E of (1RS,3RS,6RS)-6-Dimethylaminomethyl-1-(3-methoxy-phenyl)cyclohexane-1,3-diol hydrochloride from the other modifications it is more advantageous to look at the unique peaks in the X-ray diffraction diagram, i.e. the lines with sufficient intensity at 2-theta values, where the other modifications do not show lines with significant intensity. Such characteristic X-ray lines (2-theta values) for form D in a powder diffraction pattern when measured using CuK_{α} radiation at ambient temperature are: 10.7± 0.2, 14.9±0.2, 21.4±0.2, 22.4±0.2, 24.2±0.2 and 28.9±0.2

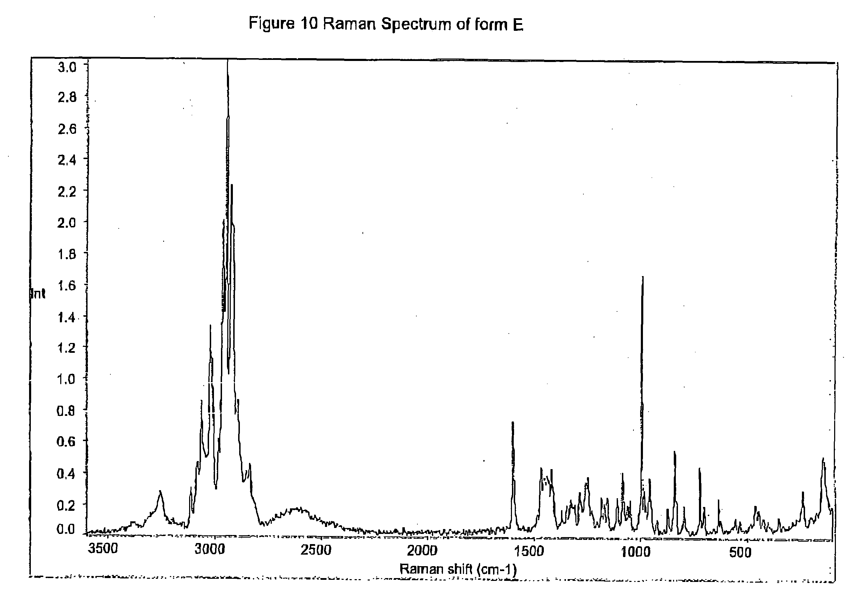

RAMAN technique can also be used to identify of the crystalline form E of (1RS,3RS,6RS)-6-Dimethylaminomethyl-1-(3-methoxy-phenyl)cyclohexane-1,3-diol hydrochloride as shown in Fig. 10.

The Invention further relates to processes for the preparation of crystalline form E of (1RS,3RS,6RS)-6-Dimethylaminomethyl-1-(3-methoxy-phenyl)cyclohexane-1,3-diol hydrochloride.

Object of the invention is a process for production of (1RS,3RS,6RS)-6-Dimethylaminomethyl-1-(3-methoxy-phenyl)cyclohexane-1,3-diol hydrochloride of crystalline form E according to claim 28-32, comprising the steps heating form B to 80-100°C ,preferred 85-95°C, for 20-40, preferred 25-35, minutes.

The very preferred process starts from the freshly prepared (1 RS,3RS,6RS)-6-Dimethytaminomethyt-1 -(3-methoxy-phenyl)cydohexane-1,3-diot hydrochloride form B. A small sample is heated in a XRPD glass capillary to 90 °C for approx. 30 minutes. At this temperature (1RS,3RS,6RS)-6-Dimethylaminomethyl-1-(3-methoxyphenyl)cyclohexane-1,3-diol hydrochloride form E was recovered. Alternatively form E is prepared by drying freshly prepared (1 RS,3RS,6RS)-6-Dimethylaminomethyl-1-(3-methoxy-phenyl)cyclohexane-1,3-diol hydrochloride form B at 60 °C at ambient relative humidity for 2 to 6 weeks.
The preferred crystalline form is form A.
The preferred crystalline form is form B.
The preferred crystalline form is form C.
The preferred crystalline form is form D.
The preferred crystalline.form is form E.

The very preferred crystalline forms are A, B, C especially preferred form is C.

Further objects of the invention are also different mixtures selected from the form A, B, C, D, E.

Further objects of the invention are compositions comprising mixtures of crystalline forms according to the invention, preferred mixtures of crystalline forms comprising one or more members selected from the group of the forms A, B, C, very preferred comprising form C.

Further objects of the invention are pharmaceutical composition containing as active ingredient a or a mixture of, preferred a, crystalline form(s) according to the invention and containing preferred at least one suitable additive and/or auxiliary substance.

Further objects of the invention are the use of a or a mixture of, preferred a, crystalline form(s) according to the invention for production of a medicament for treating pain, preferred acute and chronic pain.

Pharmaceutical compositions according to the invention contain preferred in addition to the crystalline forms (1 RS,3RS,6RS)-6-Dimethylaminomethyl-1-(3-methoxyphenyl)cyclohexane-1,3-diol hydrochloride, preferred one or more suitable additive and/or auxiliary substance such as for example carrier materials, fillers, solvents, diluents, colouring agents and/or binders, and may be administered as liquid medicament preparations in the form of injectable solutions, drops or juices, as semisolid medicament preparations in the form of granules, tablets, pellets, patches, capsules, plasters or aerosols. The choice of the auxiliary substances, etc., as well as the amounts thereof to be used depend on whether the medicament is to be administered orally, per orally, parenterally, intravenously, intraperitoneally, intradermally, intramuscularly, intranasally, buccally, rectally or topically, for example to the skin, the mucous membranes or the eyes. For oral application suitable preparations are in the form of tablets, sugar-coated pills, capsules, granules, droplets, juices and syrups, while for parenteral, topical and inhalative application suitable forms are solutions, suspensions, readily reconstitutable dry preparations, as well a& sprays. Crystalline forms in a depot form, in dissolved form or in a plaster, optionally with the addition of agents promoting skin penetration, are suitable percutaneous application preparations. Preparation forms that can be administered orally or percutaneously can provide for the delayed release of crystalline forms according to the invention. In principle further active constituents known to the person skilled in the art may be added to the medicaments according to the invention.

The amount of active constituent to be administered to the patient varies depending on the patient's weight, on the type of application, medical indication and severity of the condition. Normally 0.005 to 1000 mg/kg, preferably 0.05 to 5 mg/kg of the crystalline forms according to the invention are administered.

Preferably, the crystalline forms according to the invention are used for the treatment of pain, preferred chronic and acute pain.

Additionally the invention relates to a method of treatment using a sufficient amount of the crystalline forms according to the invention for the treatment of a disease, preferred treating pain, urinary in continence, depression, anxiety, very preferred pain, especially preferred chronic and acute pain.

The following Examples shall further illustrate the invention without limiting it thereto.

### Example 1: Powder diffraction patterns of forms A, B and C

Powder Data Collection was done with a STOE Stadi P Powder Diffractometer equipped with a curved germanium monochromator and a linear position sensitive detector. The samples were prepared as flat samples. As source of the beam a coppec X-ray tube with monochromatized Cu K_{α1} (λ = 1.54051 A) radiation generated at 50 kV and 30 mA was used. The 2θ area for the measurement was 2° - 50°. The used step width was 0.05 degrees. The data were collected at a temperature of 23 ± 1°.

The X-ray powder pattern for form A is shown in Figure 1, the X-ray powder pattern for form B is shown in Figure 3 and the X-ray powder pattern for form C is shown in Figure 5.

The data are shown in Table 1.

### Example 2: Powder diffraction patterns of forms D and E

Powder Data Collection was carried out on a Shimadzu XRD-6000 X-ray powder diffractometer using Cu Kα radiation. The instrument is equipped with a fine focus X-ray tube. The tube voltage and amperage were set to 40 kV and 40 mA, respectively. The divergence and scattering slits were set at 1° and the receiving slit was set at 0.15 mm. Diffracted radiation was detected by a Nal scintillation detector. A theta-two theta continuous scan at 3 °/min (0.4 sec/0.02° step) from 2.5 to 40 °2 θ was used. A silicon standard was analyzed each day to check the instrument alignment. The data were collected at a temperature of 23 ± 1°.

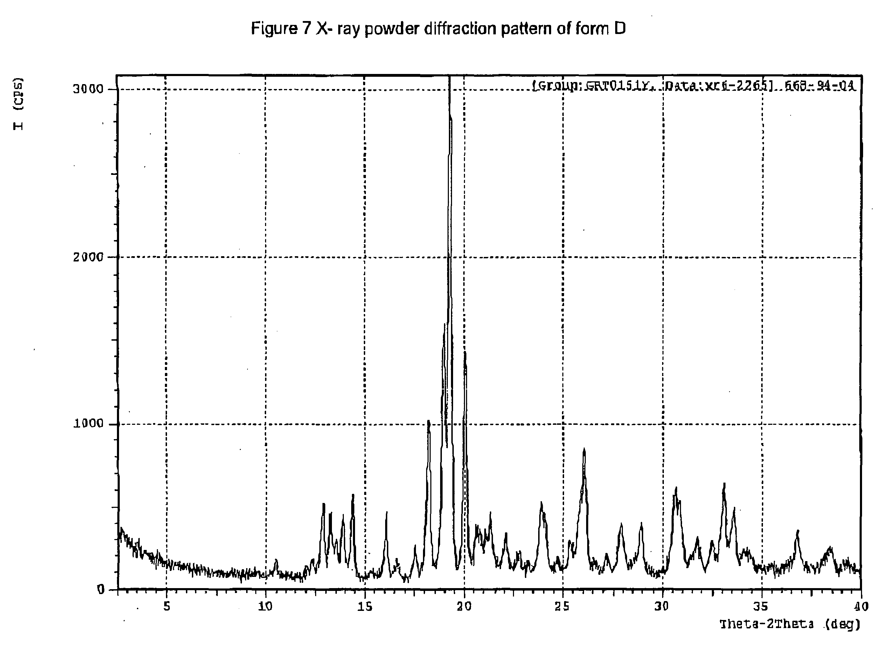

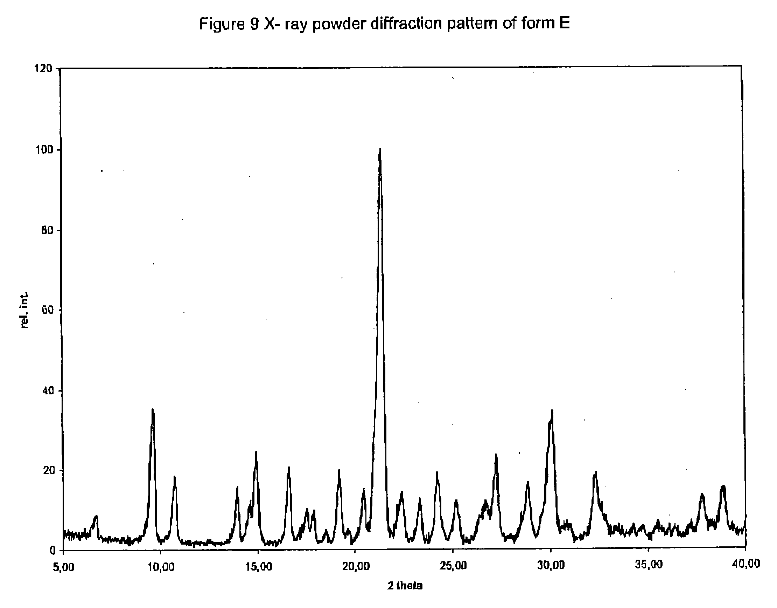

The X-ray powder diffraction pattern for form D is shown in Figure 7 and the X-ray powder diffraction pattern for form E is shown in Figure 9.

The data are shown in Table 1.

**Table 1: Peak and Relative Intensity Listing (degree 2θ, peaks with I/I1 value)**

| **Peak No.** | **A** | **I/II** | **B** | **I/I1** | **C** | **I/I1** | **D** | **I/I1** | **E** | **I/I1** |
|---|---|---|---|---|---|---|---|---|---|---|
| 1 | 12,87 | 35 | 6,43 | 29 | 6,55 | 7 | 10,28 | 9 | 6,76 | 8 |
| 2 | 13,25 | 11 | 9,67 | 46 | 11,89 | 22 | 12,74 | 21 | 9,68 | 35 |
| 3 | 14,08 | 12 | 10,52 | 30 | 12,19 | 26 | 13,02 | 26 | 10,74 | 18 |
| 4 | 14,28 | 18 | 12,57 | 17 | 12,58 | 26 | 13,54 | 18 | 13,96 | 16 |
| 5 | 15,77 | 14 | 13,61 | 64 | 12,97 | 20 | 13,7 | 21 | 14,50 | 11 |
| 6 | 16,09 | 9 | 14,24 | 42 | 14,05 | 46 | 14,1 | 20 | 14,94 | 24 |
| 7 | 16,62 | 23 | 14,64 | 70 | 14,93 | 17 | 15,78 | 21 | 16,62 | 21 |
| 8 | 17,53 | 30 | 16,34 | 52 | 15,39 | 19 | 16,24 | 10 | 17,52 | 10 |
| 9 | 17,92 | 27 | 17,05 | 23 | 15,89 | 13 | 17.5 | 15 | 17,90 | 10 |
| 10 | 18,95 | 79 | 17,54 | 58 | 16,35 | 17 | 17,94 | 40 | 19,20 | 20 |
| 11 | 19,29 | 100 | 19,34 | 20 | 17,25 | 29 | 18,6 | 66 | 20,44 | 15 |
| 12 | 20,04 | 21 | 20,36 | 29 | 17,44 | 55 | 19,04 | 100 | 21,38 | 100 |
| 13 | 20,38 | 10 | 20,64 | 100 | 18,34 | 20 | 19,90 | 50 | 23,28 | 13 |
| 14 | 21,01 | 47 | 21,06 | 83 | 19,47 | 100 | 20,4 | 18 | 24,18 | 19 |
| 15 | 21,99 | 8 | 21,61 | 40 | 19,96 | 58 | 21,1 | 17 | 25,16 | 12 |
| 16 | 23,84 | 8 | 22,06 | 33 | 21,14 | 48 | 21,92 | 10 | 25,18 | 12 |
| 17 | 24,68 | 17 | 23,17 | 23 | 22,27 | 15 | 23,58 | 23 | 25,20 | 10 |
| 18 | 25,28 | 40 | 23,56 | 23 | 22,84 | 9 | 25,3 | 19 | 25,22 | 11 |
| 19 | 25,83 | 9 | 23,92 | 15 5 | 23,37 | 40 | 25,7 | 34 | 25,24 | 11 |
| 20 | 25,99 | 10 | 24,57 | 19 | 23,85 | 16 | 27,2 | 20 | 26,70 | 12 |
| 21 | 27,20 | 7 | 25,75 | 28 | 24,44 | 25 | 27,8 | 22 | 27,26 | 24 |
| 22 | 27,76 | 12 | 26,31 | 17 | 25,22 | 16 | 28,7 | 22 | 28,86 | 17 |
| 23 | 29,38 | 11 | 26,80 | 16 | 26,15 | 14 | 30,5 | 24 | 30,12 | 34 |
| 24 | 29,85 | 13 | 27,16 | 39 | 26,58 | 47 | 31,7 | 11 | 32,34 | 19 |
| 25 | 30,64 | 12 | 28,31 | 16 | 30,02 | 21 | 32,4 | 10 | 37,80 | 13 |
| 26 | 31,34 | 9 | 29,18 | 36 | 31,04 | 15 | 33,1 | 22 | 38,94 | 15 |
| 27 | 31,83 | 10 | 29,58 | 55 | 32,16 | 10 | 33,6 | 18 | | |
| 28 | 32,38 | 6 | 31,49 | 24 | 32,58 | 16 | 36,8 | 15 | | |
| 29 | 33,02 | 16 | 31,69 | 43 | 33,44 | 16 | 38,4 | 11 | | |
| 30 | 33,50 | 16 | 32,03 | 29 | 34,98 | 12 | 10,28 | 9 | | |
| 31 | | | 34,44 | 13 | 36,98 | 8 | | | | |
| 32 | | | 36,94 | 12 | 37,29 | 10 | | | | |

### Example 3: RAMAN spectra of forms A, B and C

The polymorphs of (1RS,3RS,6RS)-6-Dimethylaminomethyl-1-(3-methoxy-phenyl)cyclohexane-1,3-diol hydrochloride were investigated using RAMAN microscopy. The RAMAN spectrometer used was a Jobin Yvon Horiba Labram. The microscope was an Olympus BX40 System, 100x Obj., diode laser 632 nm. Raman microscopy was able to distinguish between forms A, B, C and D. Differences between the spectra of the two forms appear in the whole spectral range (3500-150 cm⁻¹).

The results for form A are shown in Figure 2, the results for form B in Figure 4, the results for form C in Figure 6.

### Example 4: RAMAN spectra of forms D and E

The polymorphs of (1RS,3RS,6RS)-6-Dimethylaminomethyl-1-(3-methoxyphenyl)cydohexane.1,3.diol hydrochloride were investigated using RAMAN spectroscopy
The Raman spectrum was acquired on a Raman accessory interfaced to a Nicolet Magna 960 Fourier transform infrared spectrometer using InGaAs detector. The accessory utilizes an excitation wavelength of 1064 nm and approximately 0.45 W of Nd:YAG laser power. The spectrum represents 256 co-added scans acquired at 4 cm-1 resolution. The sample was prepared for analysis by placing a portion into a 5-mm diameter glass tube and positioning this tube in the spectrometer. The spectrometer was calibrated (wavelength) with sulfur and cyclohexane at the time of use.
The results for form D are shown in Figure 8, the results for form E in Figure 10.

### Example 5: Variable Temperature X-ray powder diffraction experiment

Variable temperature Powder Diffraction Data Collection was carried out on a Shimadzu XRD-6000 X-ray powder diffractometer using **Cu Kα** radiation. The instrument is equipped with a fine focus X-ray tube. The tube voltage and amperage were set to 40 kV and 40 mA, respectively. The divergence and scattering slits were set at 1 ° and the receiving slit was set at 0.15 mm. Diffracted radiation was detected by a Nal scintillation detector. A theta-two theta continuous scan at 3 °/min (0.4 sec/0.02° step) from 2.5 to 40 °2 θ was used. A silicon standard was analyzed each day to check the instrument alignment. The instrument was equipped with an Anton Paar HTK 1200 high temperature stage and a ceramic sample holder

A variable temperature X-ray powder diffraction experiment was run thereby producing form E from form B. form B converted to form E at 90 °C after half an hour during the experiment.

## Claims

1. Crystalline form A of (1 RS,3RS,6RS)-6-Dimethylaminomethyl-1-(3-methoxyphenyl)cyclohexane-1,3-diol hydrochloride showing at least X-ray lines (2-theta values) in a powder diffraction pattern when measured using **Cu Kα** radiation at 12.9±0.2, 17.5±0.2, 19.0±0.2, 19.3±0.2, 21.0±0.2 and 25.3±0.2.

2. Crystalline form A of (1 RS,3RS,6RS)-6-Dimethylaminomethyl-1-(3-methoxyphenyl)cyclohexane-1,3-diol hydrochloride according to claim 1 showing in addition at least X-ray lines (2-theta values) in a powder diffraction when measured using **Cu Kα** radiation at 11.0±0.2, 12.3±0.2, 12.9±0.2, 16.6±0.2, 17.9±0.2, 19.0±0.2 and 25.3±0.2.

3. Crystalline form A of (1RS,3RS,6RS)-6-Dimethylaminomethyl-1-(3-methoxyphenyl)cyclohexane-1,3-diol hydrochloride according to claim 1 showing a X-ray pattern (2-theta values) in a powder diffraction when measured using Cu Kα radiation essentially as in Fig. 1.

4. Crystalline form A of (1RS,3RS,6RS)-6-Dimethytaminomethyt-1 -(3-methoxyphenyl)cyclohexane-1,3-diol hydrochloride according to claim 1 **characterized by** RAMAN wave numbers (cm⁻¹) in a RAMAN spectrum when measured using a laser wave length of 632nm at 227 ± 4, 262 ± 4, 643 ± 4, 716 ± 4, 813 ± 4, 830 ± 4, 970 ± 4, 993 ± 4, 1252 ± 4, 2973 ± 4 and 3273 ± 4 cm⁻¹.

5. Crystalline form A of (1RS,3RS,6RS)-6-Dimethylaminomethyl-1-(3-methoxyphenyl)cyclohexane-1,3-diol hydrochloride according to claim 1 showing a RAMAN spectrum when measured using a laser wave length of 632 nm essentially as in Fig. 2.

6. A process for the production of form A according to claim 1-5.

7. Process for production of (1RS,3RS,6RS)-6-Dimethylaminomethyl-1-(3-methoxy-phenyl)cyclohexane-1,3-diol hydrochloride of crystalline form A according to claim 1-6 comprising precipitating the free base solution of (1RS,3RS,6RS)-6-Dimethylaminomethyl-1-(3-methoxy-phenyl)cyclohexane-1,3-diol hydrochloride, steering and filtering, drying of the solid at 40-60 °C, preferred at 45-55°C, and reduced pressure of less than 300 mbar, preferred 150 mbar, for 20-40, preferred 20-28, hours, keeping the temperature at 120-140°C, preferred 125-135°C ,for 60-80, preferred 70-74, hours at less than 150 mbar pressure, reducing the temperature to 50-70, preferred 55-65°C, and drying the product for another 20-60, preferred 20-30, hours at 50-70, preferred 55-65°C, at less than 150 mbar.

8. Crystalline form B of (1RS,3RS,6RS)-6-Dimethylaminomethyl-1-(3-methoxyphenyl)cyclohexane-1,3-diol hydrochloride showing at least X-ray lines-(2-theta values) in a powder diffraction pattern when measured using **Cu Kα** radiation at 9.7±0.2, 13.6±0.2, 14.6±0.2, 16.3±0.2, 20.6±0.2 and 29.6±0.2.

9. Crystalline form B of (1RS,3RS,6RS)-6-Dimethylaminomethyl-1-(3-methoxyphenyl)cyclohexane-1,3-diol hydrochloride according to claim 8 showing in addition at least X-ray lines (2-theta values) in a powder diffraction when measured using **Cu Kα** radiation at 9.7±0.2, 10.5±0.2, 13.6±0.2, 14.6±0.2, 20.6±0.2, 21.6±0.2 , 27.2±0.2 and 29.6±0.2.

10. Crystalline form B of (1RS,3RS,6RS)-6-Dimethylaminomethyl-1-(3-methoxyphenyl)cyclohexane-1,3-diol hydrochloride according to claim 8-9 showing a X-ray pattern (2-theta values) in a powder diffraction when measured using **Cu Kα** radiation essentially as in Fig. 3.

11. Crystalline form B of (1RS,3RS,6RS)-6-Dimethylaminomethyl-1 -(3-methoxyphenyl)cyclohexane-1,3-diol hydrochloride according to claim 8-10 **characterized by** RAMAN wave numbers (cm-1) in a RAMAN spectrum when measured using a laser wave length of 632nm at 633 ± 4, 720 ± 4, 836 ± 4, 995 ± 4, 1109 ± 4, 1600 t 4, 2921 ± 4 and 2944 ± 4 cm⁻¹.

12. Crystalline form B of (1RS,3RS,6RS)-6-Dimethylaminomethyl-1-(3-methoxyphenyl)cyclohexane-1,3-diol hydrochloride according to claim 8-11 showing a RAMAN spectrum when measured using a laser wave length of 632 nm essentially as in Fig. 4.

13. A process for the production of form B according to claim 8-12.

14. Process for production of (1RS,3RS,6RS)-6-Dimethylaminomethyl-1-(3-methoxy-phenyl)cyclohexane-1,3-diol hydrochloride of crystalline form B aacording claim 8-13 comprising the steps dissolving crystalline form A in ethanol and/or water, preferred ratio range ethanol to water 9,5:0,5 to 0,5 to 9,5 , very preferred ethanol and water ratio 9:1, sonication , filtering, and allowing evaporating at room temperature by atmospheric pressure.

15. Crystalline form C of (1RS,3RS,6RS)-6-Dimethylaminomethyl-1-(3-methoxyphenyl)cyclohexane-1,3-diol hydrochloride showing at least X-ray lines (2-theta values) in a powder diffraction pattern when measured using **Cu K**_{**α**} radiation at 14.1±0.2, 17.4±0.2, 19.5±0.2, 20.0±0.2, 23.4±0.2 and 26.6±0.2.

16. Crystalline form C of (1RS,3RS,6RS)-6-Dimethylaminomethyl-1-(3-methoxyphenyl)cyclohexane-1,3-diol hydrochloride according to claim 15 showing in addition at least X-ray lines (2-theta values) in a powder diffraction when measured using **Cu Kα** radiation at 11.9± 0.2, 12.2±0.2, 12.6±0.2, 15.4±0.2, 17.3±0.2, 22.3±0.2 and 23.4±0.2.

17. Crystalline form C of (1RS,3RS,6RS)-6-Dimethylaminomethyl-1-(3-methoxyphenyl)cyclohexane-1,3-diol hydrochloride according to claim 15-17 showing a X-ray pattern (2-theta values) in a powder diffraction when measured using **Cu Kα** radiation essentially as in Fig. 5.

18. Crystalline form C of (1RS,3RS,6RS)-6-Dimethylaminomethyl-1-(3-methoxyphenyl)cyclohexane-1,3-diol hydrochloride according to claim 15-17 **characterized by** RAMAN wave numbers (cm-1) in a RAMAN spectrum when measured using a laser wave length of 632nm at 239 ± 4, 305 ± 4, 448 ± 4, 502 ± 4, 537 ± 4, 722 ± 4, 830 ± 4, 992 ± 4, 1094 ± 4, 1243 ± 4 , 2928 ± 4 and 2945 ± 4 cm⁻¹.

19. Crystalline form C of (1RS,3RS,6RS)-6-Dimethylaminomethyl-1-(3-methoxyphenyl)cyclohexane-1,3-diol hydrochloride according to claim 15-18 showing a RAMAN spectrum when measured using a laser wave length of 632 nm essentially as in Fig. 6.

20. A process for the production of form C according to claim 15-19.

21. Process for production of (1RS,3RS,6RS)-6-Dimethylaminomethyl-1-(3-methoxy-phenyl)cyclohexane-1,3-diol hydrochloride of crystalline form C according to claim 15-20 comprising the steps dissolving the free base of (1 RS,3RS,6RS)-6-Dimethylaminomethyl-1-(3-methoxy-phenyl)cyclohexane-1,3-diol hydrochloride in isopropanol at 40- xx°C(boiling point of isopropanol), cooling and treatment of solution with hydrogenchloride.

22. Crystalline form D of (1RS,3RS,6RS)-6-Dimethylaminomethyl-1-(3-methoxyphenyl)cyclohexane-1,3-diol hydrochloride showing, at least X-ray lines (2-theta values) in a powder diffraction pattern when measured using **Cu Kα** radiation at 17.9±0.2, 18.6±0.2, 19.0±0.2, 19.9±0.2 and 25.7±0.2.

23. Crystalline form D of (1RS,3RS,6RS)-6-Dimethylaminomethyl-1-(3-methoxyphenyl)cyclohexane-1,3-diol hydrochloride according to claim 22 showing in addition at least X-ray lines (2-theta values) in a powder diffraction when measured using **Cu Kα** radiation at 10.3±0.2, 12.7±0.2, 13.0±0.2, 13.5±0.2, 18.6±0.2, 25.7±0.2 and 28.7±0.2.

24. Crystalline form D of (1RS,3RS,6RS)-6-Dimethylaminomethyl-1-(3-methoxyphenyl)cyclohexane-1,3-diol hydrochloride according to claim 22-23 showing a X-ray pattern (2-theta values) in a powder diffraction when measured using **Cu Kα** radiation essentially as in Fig. 7.

25. Crystalline form D of (1 RS,3RS,6RS)-6-Dimethylaminomethyl-1 -(3-methoxyphenyl)cyclohexane-1,3-diol hydrochloride according to claim 22-24 showing a RAMAN spectrum when measured using a laser wave length of 632 nm essentially as in Fig. 8.

26. A process for the production of form D according to claim 12-25.

27. Process for production of (1RS,3RS,6RS)-6-Dimethylaminomethyl-1-(3-methoxy-phenyl)cyclohexane-1,3-diol hydrochloride of crystalline form D according to claim 22-26 comprising heating form B to 160-185°C, preferred 175-185°C, for 20-50, preferred 30-40 minutes, and cooling to room temperature.

28. Crystalline form E of (1RS,3RS,6RS)-6-Dimethylaminomethyl-1-(3-methoxyphenyl)cyclohexane-1,3-diol hydrochloride showing at least X-ray lines (2-theta values) in a powder diffraction pattern when measured using Cu K_{α} radiation at 9.7±0.2, 14.9±0.2, 16.6±0.2, 19.2±0.2, 21.4±0.2 and 27.3±0.2.

29. Crystalline form E of (1RS,3RS,6RS)-6-Dimethylaminomethyl-1-(3-methoxy-phenyocyclohexane-1,3-diol hydrochloride according to claim 28 showing in addition at least X-ray lines (2-theta values) in a powder diffraction when measured using **Cu Kα** radiation at 10.7±0.2, 14.9±0.2, 21.4±0.2, 22.4±0.2, 24.2±0.2 and 28.9±0.2.

30. Crystalline form E of (1RS,3RS,6RS)-6-Dimethylaminomethyl-1-(3-methoxyphenyl)cyclohexane-1,3-diol hydrochloride according to claim 28-29 showing a X-ray pattern (2-theta values) in a powder diffraction when measured using **Cu Kα** radiation essentially as in Fig. 9.

31. Crystalline form E of (1RS,3RS,6RS)-6-Dimethylaminomethyl-1-(3-methoxyphenyl)cyclohexane-1,3-diol hydrochloride according to claim 28-30 showing a RAMAN spectrum when measured using a laser wave length of 632 nm essentially as in Fig. 10.

32. A process for the production of form E according to claim 28-31.

33. Process for production of (1RS,3RS,6RS)-6-Dimethylaminomethyl-1-(3-methoxy-phenyl)cyclohexane-1,3-diol hydrochloride of crystalline form E according to claim 28-32, comprising the steps heating form B to 80-100°C ,preferred 85-95°C, for 20-40, preferred 25-35, minutes.

34. Composition comprising mixtures of crystalline forms according claim 1-33, preferred mixtures of crystalline forms comprising one or more members selected from the group of the forms A, B, C, preferred comprising form A.

35. Pharmaceutical composition containing as active ingredient a or a mixture of, preferred a, crystalline form(s) according to claim 1-34 and containing preferred at least one suitable additive and/or auxiliary substance.

36. Use of a or a mixture of, preferred a, form(s) according to claim 1-35 for production of a medicament for treating pain, urinary in continence, depression, anxiety, preferred pain, very preferred acute and chronic pain.
